# EUROPEAN PATENT SPECIFICATION

(11) **EP 0 787 207 B1**
(45) Date of publication and mention of the grant of the patent: **26.10.2005**
(21) Application number: 95941352.7
(22) Date of filing: 01.11.1995
(51) Int. Cl.: C12Q 1/68, G01N 33/53, G01N 33/50, G01N 33/566, G01N 33/68, G01N 33/74

(54) **NEW METHOD FOR IDENTIFYING AND EVALUATING BIOLOGICALLY ACTIVE MOLECULES**
NEUE METHODE ZUM AUFFINDENUND BEWERTEN BIOLOGISCH AKTIVER MOLEKÜLE
NOUVELLE METHODE D'IDENTIFICATION ET D'EXAMEN DE MOLECULES A ACTIVITE BIOLOGIQUE

(30) Priority: 01.11.1994 US 332995; 07.03.1995 US 400800; 07.06.1995 US 480286
(43) Date of publication of application: 06.08.1997
(73) Proprietor: ARIAD GENE THERAPEUTICS, INC., Cambridge, MA 02139 (US)
(72) Inventor: SCHREIBER, Stuart L., Boston, MA 02115 (US); CRABTREE, Gerald R., Woodside, CA 94062 (US); HOLT, Dennis A., Stow, MA 01775 (US); ZOLLER, Mark J., Weston, MA 02193 (US)
(74) Representative: Mercer, Christopher Paul
(86) International application number: PCT/US1995/014177
(87) International publication number: WO 1996/013613

(56) References cited:
- BIOLOGICAL ABSTRACTS, Philadelphia PA USA; abstract no. PREV199396122379, *abstract* XP002116983 & A.C. GREENLUND ET AL.: "Interferon-gamma induces receptor dimerization in solution and cell." JOURNAL OF BIOLOGICAL CHEMISTRY, vol. 268, no. 24, 1993, pages 18103-18110, Rockville MD USA
- CHEMICAL ABSTRACTS, vol. 129, no. 7, 17 August 1998 (1998-08-17) Columbus, Ohio, US; abstract no. 77158, XP002116984 & V.M. RIVERA: "Controlling gene expression using synthetic ligands." METHODS (ORLANDO FL), vol. 14, no. 4, 1998, pages 421-429, New York NY USA
- JOURNAL BIOLOGICAL CHEMISTRY, Volume 266, No. 30, issued 25 October 1991, HEIDARAN et al., "Role of Ab Receptor Heterodimer Formation in B Platelet-derived Growth Factor (PDGF) Receptor Activation by PDGF-AB", pages 20232-20237.
- MOLECULAR AND CELLULAR BIOLOGY, Volume 12, issued January 1992, ORNITZ et al., "Heparin is Required for Cell-free Binding of Basic Fibroblast Growth Factor to a Soluble Receptor and for Mitogenesis in Whole Cells", pages 240-247.
- SCIENCE, Volume 262, issued 12 November 1993, SPENCER et al., "Controlling Signal Transduction With Synthetic Ligands", pages 1019-1024.
- CHEMISTRY & BIOLOGY, Volume 1, No. 3, issued 1994, PRUSCHY et al., "Mechanistic Studies of a Signaling Pathway Activated by the Organic Dimerizer FK1012", pages 163-172.
- PROC. NATL. ACAD. SCI. U.S.A., Volume 92, issued October 1995, HOLSINGER et al., "Signal Transduction in T Lymphocytes Using a Conditional Allele of Sos", pages 9810-9814.
- PROC. NATL. ACAD. SCI. U.S.A., Volume 92, issued October 1995, SPENCER et al., "A General Strategy for Producing Conditional Alleles of Src-like Tyrosine Kinases", pages 9805-9809.
- BIOCHEMISTRY, Volume 30, issued 1991, "Ligand-induced Interaction Between A- and B-type Platelet-derived Growth Factor (PDGF) Receptors: Role of Receptor Heterodimers in Kinase Activation", pages 1761-1767.
- CHEMISTRY & BIOLOGY, Volume 1, issued 1994, AUSTIN et al., "Proximity Versus Allostery: the Role of Regulated Protein Dimerization in Biology", pages 131-136.

## Description

### Technical Field

This invention concerns materials, methods and applications thereof relating to the multimerizing of protein mediators of biological events using synthetic, preferably non-peptidic, dimerizing agents.

### Background

A considerable amount of research has been directed to the identification and characterization of protein-protein interactions involved in mediating a variety of biological events. A growing number of research groups in academic and industrial laboratories have focused their efforts on inhibiting certain protein-protein interactions which are believed to mediate disease processes. Other research has focused in part on dimerization of chimeric receptors. Aspects of that work are disclosed in Spencer et al, 12 November 1993, Science 262:1019-1024 and WO 94/18317 and WO 95/02684.

The subject invention is a departure from the foregoing efforts in several respects. It involves promoting interactions between endogenous proteins which mediate a desired biological event. By "endogenous" proteins, as the phrase is used herein, we refer to proteins originating on or within a cell or invading organism thereof-as opposed to "chimeric" proteins resulting from the expression of a recombinant DNA sequence.

### Description of the Invention

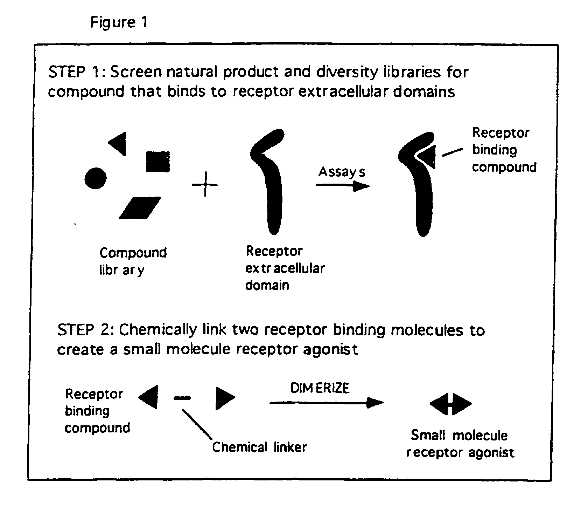

This invention provides a method for identifying an agent capable of effecting a biological event in a cell, which event is (or can be) mediated by the association of two or more protein mediators, at least one, and usually both, of which are endogenous. The method involves the steps of identifying a first compound capable of binding to one of the protein mediators and a second compound capable of binding to the other protein mediator. The two compounds are then covalently joined to one another to form a chemical inducer of dimerization (CID) which is capable of binding to both mediators as depicted schematically in Figure 1. Assay methods are provided for identifying the monomeric binding compounds and for evaluating and optimizing the CIDs produced from them.

In embodiments in which the biological event of interest is mediated by association of two or more copies of the same mediator species, e.g. a receptor for a cytokine, growth factor or other hormone, the first and second compounds may be the same compound. In such cases the CID is typically a homodimer of that compound. Examples of such mediators include transcription factors such as the STAT-91 protein and receptors for polypeptide growth factors and hormones such as those illustrated in Table I:

**Table I**

| **Mediator** | **Receptor Type** | **Ligand** | **Therapeutic Application** |
|---|---|---|---|
| EPO receptor | Class I Cytokine | EPO | Anemia |
| G-CSF receptor | Class ICytokine | G-CSF | Neutropenia |
| TPO receptor (c-Mpl) | Class I Cytokine | TPO | Thrombocytopenia |
| GH receptor | Class I Cytokine | GH | GH deficiency |
| IL-2 receptor | Class I Cytokine | IL-2 | Cancer |
| IFN-alpha receptor | Class II Cytokine | IFN-alpha | Hepatitis C |
| IFN-beta receptor | Class II Cytokine | IFN-beta | Multiple Sclerosis |
| Insulin receptor | Tyrosine kinase | Insulin | Diabetes |
| Trk receptors | Tyrosine kinase | NTFs | CNS diseases |

Polypeptide growth factors, cytokines and hormones, such as insulin, erythropoietin (EPO), growth hormone (GH) and granulocyte colony stimulating factor (G-CSF) activate intracellular processes upon binding to specific cell surface receptors (Ullrich, A. and Schlessinger, J., "Signal transduction by receptors with tyrosine kinase activity", Cell, 61: 203-212 (1990); Kishimoto, T., Taga, T., and Akira, S., "Cytokine signal transduction", Cell, 76:253-262 (1994)). These receptors are composed of three domains: an extracellular ligand binding domain, a transmembrane domain, and an intracellular signal transduction domain. Some receptors, such as those for GH and EPO, have the ligand binding domain and signaling domain on the same polypeptide. Others, such as receptors for IL-3 and IL-6, have separate ligand binding and signal transduction subunits. It is now clear that signal transduction by cytokines and growth factors is accomplished by ligand-mediated receptor dimerization (Heldin, C. H., "Dimerization of cell surface receptors in signal transduction", Cell, 80: 213-223 (1995); Lemmon, M.A., and Schlessinger, J., "Regulation of signal transduction and signal diversity by receptor oligomerization", Trends Biol. Sci., 19:459-463 (1994)). For example, EGF binds to two receptor subunits resulting in dimerization of the cytoplasmic tyrosine kinase domains. This association of intracellular domains stimulates the tyrosine kinase activity and initiates a cascade of intracellular processes. Recent work on cytokine receptors has demonstrated that their signal transduction is also mediated by receptor dimerization (Murakami, M., et al, "IL-6-induced homodimerization of gp130 and associated activation of a tyrosine kinase", Science, 260:1808-1810 (1993)).

Unlike G protein-coupled receptors where precise ligand-induced conformational changes are required for initiation of signaling events, receptors that are activated by dimerization require only imprecise aggregation of their cytoplasmic domains. For example, cells expressing an EPO receptor variant containing an additional extracellular cysteine were constitutively activated in the absence of EPO by the formation of disulfide linked receptor dimers (Watowich, et al, "Homodimerization and constitutive activation of the erythropoietin receptor", Proc. Natl. Acad. Sci. USA. 89:2140-2144 (1992)). In other studies, bivalent antibodies to growth hormone receptor dimerized the receptor subunits and activated GH-mediated cell proliferation (Fuh, G., et al, "Rational design of potent antagonists to the human growth hormone receptor", Science, 256:1677-1680 (1992)).

In embodiments in which the biological event of interest is mediated by association of two or more different mediator proteins, the first and second component compounds of a CID will usually be two different compounds. Examples of biological events which can be mediated by the association of different mediator proteins include transcriptional activation (mediated by association of a protein containing a DNA-binding domain with a protein containing a transcriptional activation domain), the targeting of a protein to a particular location (mediated by association of the protein to be targeted with a targeting protein), including the targeting of a protein for degradation via the proteosome, etc.

Where the mediators of the biological event of interest are present within a cell, contacting such cells with an amount of the CID effective to result in association of the mediator protein(s) results in the occurrence of the biological event of interest, e.g. in gene transcription, protein localization, receptor signalling, etc. Contacting the cells with the dimerizing agent is effected by adding the CID to the culture medium in which the cells are growing, or, if the cells are or may be present within an organism, by administration of the CID to the organism. The organism may be plant or animal, and in the latter case may be an insect, mammal (including among others, rodents such as mice and rats, and primates, including humans) or other animal. In cases in which the CID is administered to an animal or human, it may be administered in the form of a veterinary or pharmaceutical composition containing the CID and one or more suitable diluents, carriers, adjuvants and the like, as are well known in the art. Such compositions may contain conventional carriers for the various modes of administration including oral and parenteral administration. Additional guidance on such compositions and their administration may be adapted from the details disclosed in WO 94/18317 (see especially pp. 46-48).

### Mediators & Biological Events

Cellular processes which can be triggered by oligomerization include a change in state, such as a physical state, *e.g*. conformational change, change in binding partner, cell death, initiation of transcription, channel opening, ion release, *e.g*. Ca⁺² *etc.* or a chemical state, such as a chemical reaction, *e.g*. acylation, methylation, hydrolysis, phosphorylation or dephosphorylation, change in redox state, rearrangement, or the like. Any such process which can be triggered by the association or oligomerization of endogenous cellular constituents is within the scope of this invention, including for example, signaling triggered by the association of mediators such as growth factor receptors and the "forwarding" of one endogenous constituent to the cellular environment or fate of another endogenous constituent using a dimerizing agent capable of binding to both constituents.

### Compounds capable of binding to the mediators: characteristics & methods for obtaining them

Many compounds capable of binding to a variety of protein mediators of biological events are already known. For instance, many benzodiazepines, prostaglandins, beta-turn mimetics, alpha- and beta-blockers, FK506 (and related compounds such as rapamycin and their analogs), steroids, retinoids, topoisomerase inhibitors and other ligands which bind to their respective receptors or binding partners are known. Other compounds capable of binding to those receptors or to other endogenous constituents may be readily identified using a variety of approaches, including phage display and other biological approaches for identifying peptidyl binding compounds; synthetic diversity or combinatorial approaches (see e.g. Gordon et al, 1994, J Med Chem 37(9):1233-1251 and 37(10):1385-1401); and DeWitt et al, 1993, PNAS USA 90:6909-6913) and conventional screening or synthetic programs. Unlike programs to design or screen for biologically active compounds such as enzyme inhibitors or receptor agonists or antagonists, binding compounds for use in the subject invention may, but need not, bind to the mediator in a precise fashion required to inhibit, agonize or antagonize-they need only *bind* to the mediator. Compounds capable of binding to the protein of interest may be identified by various methods of affinity purification or by direct or competitive binding assays, including assays involving the binding of the protein to compounds immobilized on solid supports such as pins, beads, chips, etc.). See e.g. Gordon et al, supra.

For example, a known ligand for a receptor may be used as follows to identify compounds which bind to the ligand's receptor which may be used in CIDs of this invention. Generically stated, the method of this embodiment employs: (1) a peptide which contains a ligand-binding domain of a receptor of interest (which may be intact receptor, the ligand-binding domain thereof or a fusion protein containing the ligand-binding domain of the receptor fused to heterologous protein sequence, collectively referred to as "receptor" in the following discussion), (2) a ligand for the receptor which is capable of selectively binding to the receptor to form a ligand-receptor complex and (3) a compound (the "test substance") to be evaluated for its ability to bind competitively to the receptor. The method is carried out by combining the three components mentioned above, or compositions comprising them; incubating the resulting test mixture under conditions permitting the formation of a ligand-receptor complex; and measuring the ability of the test substance to compete with the ligand for binding to the receptor or to otherwise block the formation or reduce the observed level of receptor-ligand complex. This method is a powerful and general method, and should be applicable to any receptor-ligand pair and susceptible to variety of configurations, including both *in vitro* and *in vivo* formats. Depending on the specific assay configuration, it may be important to use known concentrations of receptor, ligand and/or test substance. For comparative purposes, the assay may also be carried out in the absence of the test substance or in the presence of varying concentrations of test substance. One may carry out the measuring step by assaying for receptor-ligand complex, non-complexed receptor and/or non/complexed test substance or by measuring the occurrence of an event mediated by the presence or formation of the receptor-ligand complex or a receptor-test substance complex. For example, in one embodiment, a ligand for a receptor is immobilized and incubated, under conditions permitting receptor-ligand binding, with a labeled receptor, or labeled peptide containing the ligand-binding domain of the receptor, in the presence and absence of a test substance or composition containing a test substance. The presence of a test substance which competes with ligand for receptor binding correlates with a decrease in labeled receptor (or labeled domain) bound to the immobilized ligand, or with an increase in unbound labeled receptor (or labeled domain). Various labels suitable for such purposes are well known in the art and may be selected based on factors such as cost, availability, convenience and familiarity on the part of the practitioner.

The test substance may be present in a solution, referred to as a test solution. Alternatively, especially for *in vitro* assays, the test substance may be present in a test mixture comprising an emulsion, suspension or other mixture; exposed on the surface of a cell, virus, phage, *etc.;* or immobilized on a solid support.

In an *in vitro* format, a binding assay is conducted to identify a compound capable of binding to the receptor in the presence of a ligand for that receptor or otherwise capable of blocking the formation or reducing the observed level of receptor-ligand complex. In one embodiment, the binding assay is a competitive binding assay in which the three components are combined and incubated under conditions permitting the formation of an receptor-ligand complex. The ability of the test substance to bind to the selected receptor or otherwise block the receptor-mediated interaction in the presence of the receptor's ligand is determined.

Binding to the receptor or otherwise blocking the receptor-mediated interaction may be measured directly or indirectly (*e.g*., BIAcore® and other SPR technologies (*BIAtechnology Handbook,* Pharmacia Biosensor AB, Uppsala, Sweden, 1994), fluorescence anisotropy and allied technologies (Luminescent Spectroscopy of Proteins, 164pp, E. A. Permyakov, CRC Press, Inc, Boca Raton, FL, 1992), flow cytometry and allied technologies (*Flow Cytometry and Cell Sorting,* 223pp., A. Radbruch, ed., Springer-Verlag, New York, NY, 1992), ELISA, RIA and allied methodologies (*An Introduction to Radioimmunoassays and Related Techniques,* 290 pp., T. Chard, Elsevier Science Publishers, Amsterdam, The Netherlands, 1990), competitive and non-competitive affinity interactions (*Immobilized Affinity Ligand Techniques,* 454 pp., G. T. Hermanson, A. K. Mallia and P.K. Smith, eds., Academic Press, Inc., San Diego, CA, 1992).

In competitive binding assays, if binding of the receptor and its ligand occurs to a lesser extent in the presence of the test substance than in its absence, for instance, if the presence of the test substance reduces the concentration of receptor-ligand complex or increases the concentration of non-complexed (i.e., to each other) receptor or ligand, then the test substance is a receptor-binding agent. If the structure of the binding agent so identified is not yet known, the compound may then be isolated from the other assay components and characterized. It may be re-evaluated, if desired, using similar binding assays with different receptor-ligand pairs to confirm the selectivity of the interaction with the receptor with which it was identified. If desired, the binding of the binding agent to the receptor with which it was identified may be characterized biochemically, *e.g*. through the use of BIAcore® technology, described in greater detail below. The binding agent so identified may be assayed in an *in vivo* assay as described below and may further be evaluated in monomer and/or CID form for pharmacological activity in various *in vitro* and/or *in vivo* assays, as desired.

*In vivo* assays can be conducted in analogous manner using cells containing the ligand-binding domain of interest and a ligand therefor. The cells are cultured or maintained in a medium suitable for cell growth. The test substance is added to the cells, *e.g.* to the medium in which the cells are cultured, and the culture is incubated under conditions permitting formation of a complex between the receptor and its ligand. If binding of the receptor and its ligand occurs to a lesser extent in the presence of the test substance than in its absence, for instance, if the presence of the test substance reduces the concentration of receptor-ligand complex or increases the concentration of non-complexed ligand, then the test substance is an binding agent. The presence or absence of receptor-ligand complex may be measured directly or indirectly (*e.g*., by measuring the occurrence of an event mediated by the presence or formation of the receptor-ligand complex or a receptor-test substance complex).

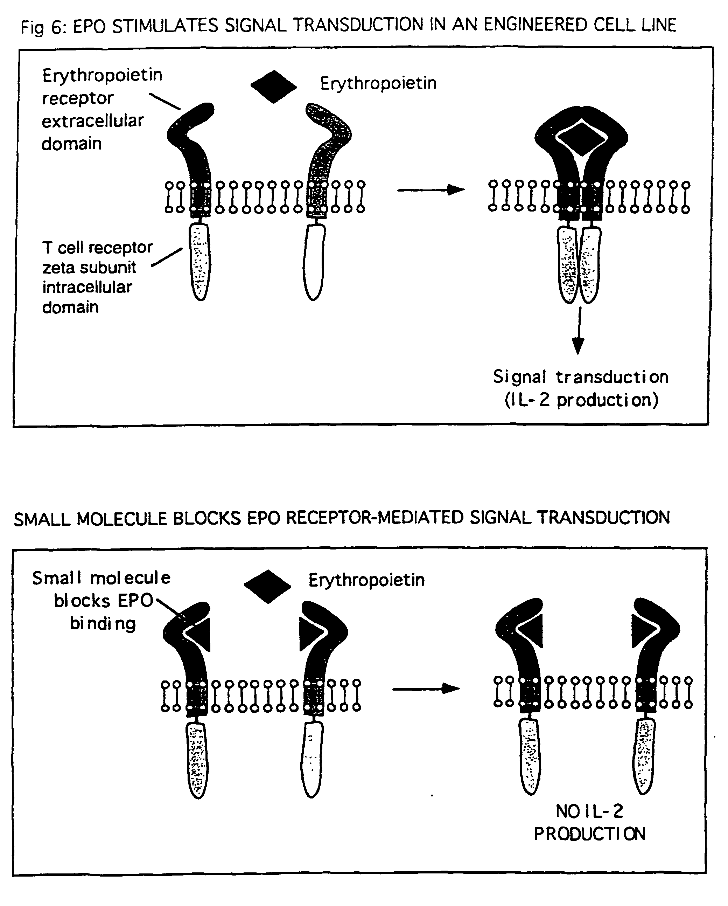

An illustrative *in vivo* format relies upon genetically engineered cells capable of expressing a reporter gene under receptor-mediated transcriptional control. These cells contain and are capable of expressing recombinant DNAs encoding a fusion protein comprising, among other component regions, at least one ligand-binding domain of the receptor of interest. The fusion proteins are capable of binding to the ligand for the receptor and in the presence of the ligand are capable of forming a complex (dimerizing) with each other as illustrated in Figure 6. In the presence of the ligand, e.g. when maintained in culture medium containing ligand, the cells express the reporter gene-unless a substance is present which binds to the receptor domain or otherwise blocks the association of the fusion proteins required for transcription of the reporter gene. In this assay, the cells are cultured or maintained in a suitable culture medium to which a selected amount of ligand is added to establish a base-line for expression of the reporter gene. The test substance is then added to the culture medium and the ability of the test substance to inhibit expression of the reporter gene is measured. If the level of reporter gene expression is reduced in the presence of the test substance, the test substance is a blocker with respect to the chimeric receptor molecules involved in transcriptional control. If the structure of the blocking agent so identified is not yet known, the compound may then be isolated from the other assay components and characterized. It may be re-evaluated, if desired, using engineered cells containing a fusion protein based on a different receptor, in medium containing ligand for that receptor, to confirm the selectivity of the interaction with the receptor domain with which it was identified. If desired, the binding affinity of the blocking agent for the receptor with which it was identified may be determined, *e.g*. such as through the use of BIAcore® technology. The blocking agent so identified may be assayed in an *in vitro* binding assay as described above and may further be evaluated for pharmacological activity in various *in vitro* and/or *in vivo* assays, as desired, again in monomer and/or CID (i.e., dimerized) form.

Binding agents identified by such methods for use in constructing CIDs of this invention can be identified from peptide libraries as well as from test substances obtained from a wide variety of sources including, *e.g*., microbial broths; cellular extracts; conditioned media from cell lines or from host cells transformed with genetic libraries; collections of synthetic compounds; combinatorial libraries or synthetic programs based on conventional medicinal chemistry approaches or structure-based drug design.

This invention thus provides a means for identifying selective binding or blocking agents. Compounds so identified may be covalently joined together using linker moieties, e.g. by adaptation of the approaches disclosed in WO 94/18317 and WO 95/02864 to form the CIDs of this invention. Linker moieties need not contain essential elements for binding to the mediators of interest, and may be selected from a very broad range of structural types. Preferred moieties include C₂-C₂₀ alkyl, aryl, or dialkylaryl structures.

Alkyl is intended to include both saturated and unsaturated straight chain, branched, cyclic, or polycyclic aliphatic hydrocarbons which may contain oxygen, sulfur, or nitrogen in place of one or more carbon atoms, and which are optionally substituted with one or more functional groups selected from the group consisting of hydroxy, C₁-C₈ alkoxy, acyloxy, carbamoyl, amino, N-acylamino, ketone, halogen, cyano, carboxyl, and aryl (unless otherwise specified, the alkyl, alkoxy and acyl groups preferably contain 1-6 contiguous aliphatic carbon atoms).

Aryl is intended to include stable cyclic, heterocyclic, polycyclic, and polyheterocyclic unsaturated C₃-C₁₄ moieties, exemplified but not limited to phenyl, biphenyl, naphthyl, pyridyl, furyl, thiophenyl, imidazoyl, pyrimidinyl, and oxazoyl; which may further be substituted with one to five members selected from the group consisting of hydroxy, C₁-C₈ alkoxy, C₁-C₈ branched or straight-chain alkyl, acyloxy, carbamoyl, amino, N-acylamino, nitro, halogen, trifluoromethyl, cyano, and carboxyl (see e.g. Katritzky, Handbook of Heterocyclic Chemistry).

Linker moieties may be conveniently joined to the binding moieties through functional groups such as ethers, amides, ureas, carbamates, and esters; or through alkyl-alkyl, alkyl-aryl, or aryl-aryl carbon-carbon bonds. Furthermore, linker moieties may be optimized (e.g., by modification of chain length and/or substituents) to enhance pharmacokinetic properties of the multimerizing agent. In cases in which the compounds are identified while immobilized, they may be conveniently linked using the functional groups by which they had been immobilized. Peptidyl compounds may be linked by peptide bonds, although the preferred agents of this invention are not polypeptides or oligopeptides. Divalent dimerizing agents of this invention retain binding capability with respect to both of the proteins of interest. Thus, the covalently linked CIDs will usually be tested (e.g. as above) to confirm retention of binding capability with respect to each of the proteins of interest and/or in cell-based assays as described below.

We note that in the design of CIDs, selecting compounds which bind to the proteins of interest from combinatorial libraries immobilized on solid supports such as beads provides a useful advantage. While this still entails identifying a new ligand for the protein of interest rather than selecting a previously known compound, the very act of discovering tells precisely how one may attach the covalent tether to link the two compounds together to form the CID. That is so because the members of an immobilized combinatorial library are already covalently linked to their support. Thus, the same chemistry may be used in assembling the CID as was used to immobilize the individual members of the library. Furthermore, since the selected library members are selected for their ability to bind to their respective receptors (or other protein binding partners), by necessity, the linker covalently attaching the library member to the support must not interfere with the binding interaction between the library member and the protein of interest.

### Assays for Functional Evaluation of CIDs

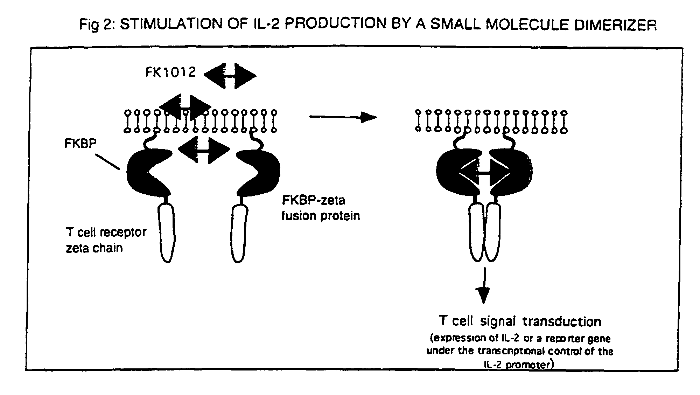

We also provide general cell-based assay methods for functional characterization of CIDs. These assays are based on cells genetically engineered in accordance with the system described in WO 94/18317. The cells are engineered to contain and be capable of expressing recombinant DNAs encoding chimeric proteins capable, upon their association or dimerization, of activating the transcription, directly or indirectly, of a reporter gene under the transcriptional control of a promoter, enhancer or other transcriptional regulatory element, responsive to the association of the chimeric proteins. Suitable materials, methods and design and construction principles for relevant constructs and their use are disclosed in WO 94/18317 and may be adapted for use in the practice of this invention as illustrated by the following example. In one embodiment, Jurkat cells are genetically engineered to contain a reporter gene such as secreted alkaline phosphatase (although any conveniently detected reporter may be used, including beta-galactosidase or luciferase for example) under the expression control of the NF-AT system, details for which are provided in the above-mentioned PCT document. Those cells disclosed in the PCT document were further engineered to contain and express a recombinant DNA sequence encoding a chimeric protein comprising a myristoylation signal, the cytoplasmic tail of the zeta chain of the T cell receptor and one or more ligand-binding domains derived from FKBP12. The cells of our assay are prepared analogously, but express one or more chimeric proteins containing, in place of the FKBP12 domain, part or all of the protein mediator of interest. Where dimerization of two different protein mediators is of interest, the cells are engineered to express a chimeric protein, as described above, corresponding to each protein mediator of interest. The presence of a CID which is capable of binding to two molecules of the chimeric protein(s) induces association or dimerization of the chimeric proteins. Such dimerization triggers a transcriptional activation signal which is received by the transcriptional control elements for the reporter gene and is readily detected by measuring the expression of the reporter molecule as depicted schematically in Figure 2. Full details and general guidance for assembling such constructs, engineering the cells and detecting the reporter molecule are provided in the PCT document. See e.g. Figs. 14,15, and 18-21 and corresponding examples therein. Again, adapting that system to provide cells for assaying CIDs of this invention is readily accomplished by replacing DNA sequence(s) encoding the FKBP domains with DNA sequence encoding the protein mediator of interest (for example the intracellular or extracellular domain of the receptor for insulin or erythropoietin, or other growth factor), taking care that the full coding region of each resultant construct is in frame.

Using such engineered cells one may functionally characterize CIDs of this invention by growing the engineered cells in culture, exposing them to the CID(s) of interest by adding an amount (usually a predetermined amount) of the CID of interest to the culture medium, and detecting the amount of reporter produced in response to the CID. Candidate CIDs containing one or more structural variations in their component binding moieties or linking moiety may be comparatively evaluated and CIDs for particular applications may thus be optimized.

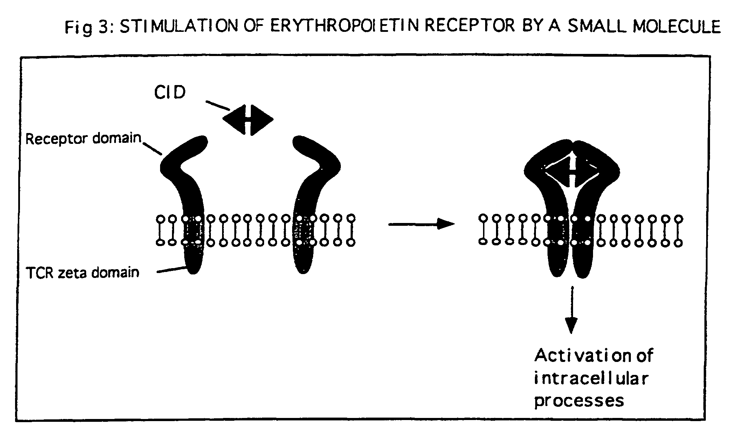

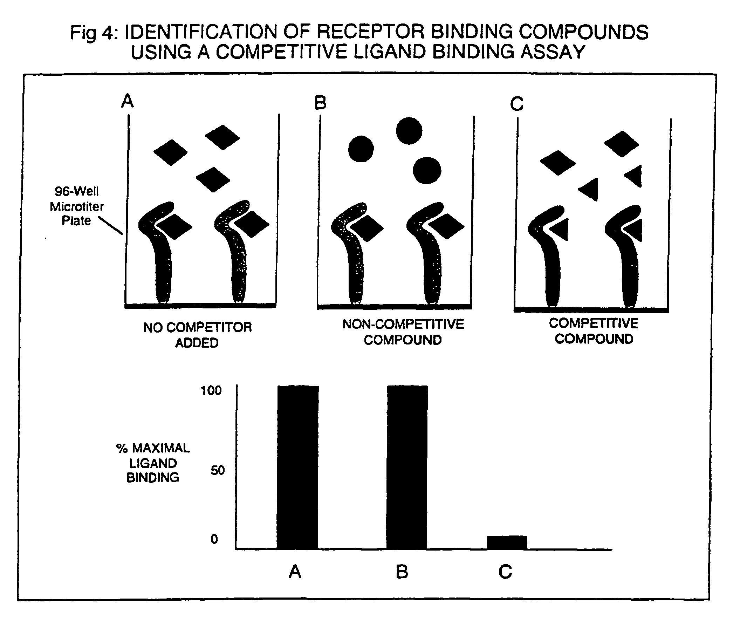

As an alternative approach to genetically engineered cells for such assay purposes, one may use a modified design for the chimeric "receptors" which will bind to the CIDs and trigger transcription of the reporter gene. In this approach the chimeric receptors contain a signaling moiety such as the zeta chain as above, a membrane spanning domain, and, as an extracellular domain, one or more copies of a domain corresponding to the protein mediator of interest of the chimeric protein(s). The assay may be conducted as described above. However, in this modification, CIDs are detected extracellularly rather than intracellularly, as depicted schematically in Figure 3. This approach will usually be preferred for evaluating peptidyl or other CIDs which do not readily enter the cells.

As mentioned at the outset, a wide variety of receptor/ligand pairs are involved in a number of pharmacologically significant events including anemia, neutropenia, thrombocytopenia, cancer, MS, diabetes, CNS disorders, etc and their treatment. Accordingly, CIDs of this invention may be useful for a variety of clinically important purpose as well as for research purposes to probe the biology of receptor-mediated phenomena.

Generally speaking, CIDs of this invention can be used to promote the occurrence of biological events resulting from molecular interactions mediated by a receptor of interest. This invention thus provides a method and reagents for promoting the interaction between endogenous proteins and thus for promoting a biological activity mediated by such interaction. In this method, a CID of this invention is combined or contacted with the receptor of interest, such as by introducing the CID into a cell in which the receptor-mediated interaction is to be promoted. Following introduction of the CID, the mutual association or dimerization of the endogenous protein to which the CID binds is promoted, as may be readily detected. Promoting such interactions can be useful in research aimed at better understanding the biology of receptor-mediated events.

Such CIDs would be useful, for example, in the diagnosis, prevention or treatment of conditions or diseases which may be cured, or have their symptoms alleviated in whole or part, by the occurrence of cellular processes mediated by a receptor-mediated interaction. For example, a patient can be treated to prevent or alleviate the occurrence or progression of anemia, thrombocytopenia, or neutropenia by the administration of a CID capable of promoting dimerization of receptor molecules for EPO, TPO or G-CSF, respectively.

A CID of this invention can be formulated into a pharmaceutical composition containing a pharmaceutically acceptable carrier and/or other excipient(s) using conventional materials and means. Such a composition can be administered to an animal, either human or non-human, for therapy of a disease or condition responsive to the promotion of cellular events involving the mutual interaction of endogenous protein molecules. Administration of such composition may be by any conventional route (parenteral, oral, inhalation, and the like) using appropriate formulations as are well known in this art. The CID can be employed in admixture with conventional excipients, ie, pharmaceutically acceptable organic or inorganic carrier substances suitable for parenteral administration.

This invention is not to be limited in scope by the specific illustrative embodiments described herein. Indeed, various modifications of the invention in addition to those described herein will become apparent to those skilled in the art from the description herein. Such modifications are intended to fall within the scope of the appended claims.Various publications are cited herein, the disclosures of which are incorporated by reference in their entireties.

### Examples

### Example 1. Enhancing the activity of known drugs or newly selected compounds, or imparting an activity, by incorporation into a CID

In this approach, a protein which functions substantially in only one cellular compartment, e.g. the cytoplasm, is diverted through binding to an appropriate CID to an alternative cellular compartment where it lacks bioactivity.

To design such a CID, one selects a first compound capable of binding to the protein target. Examples of protein targets that function only in the cytoplasm and not in the nucleus, for example, include HIV protease and various signaling proteins such as zap70, syk and the like. In the case of HIV protease, cell permeable HIV protease inhibitors have been developed by a number of groups and are known in the literature. See e.g. Lam et al, 1994, Science 263(5145): 380-4 and WO 94/08977.

One further selects a second compound which binds to a constituent of the alternative cellular compartment. Again, localization of the target protein in the alternative compartment is inconsistent with biological function of the target protein. Where the alternative compartment is the nucleus, relevant constituents include the topoisomerases to which etoposide, camptothecin and related compounds bind. The synthesis of Camptothecin and analogs thereof is known, as is their evaluation as inhibitors of topoisomerase I. See e.g. Corey et al, 1975, J Org Chem 40:2140 (total synthesis); Sugimori et al, 1994, J. Med. Chem. 37(19), 3033-9 (illustrative analogs); Prost et al, 1994, Biochem. Pharmacol. 48(5), 975-84 (experiments with topo I). Alternate nuclear targets include DNA, for which numerous intercalating agents are known. Alternative targets for directing a target protein to the mitochondria include cytochromes which are present only in that compartment.

The first and second compounds may be selected from known compounds capable of binding to the respective proteins or may be selected from combinatorial libraries as discussed above. In either event, they are then covalently joined through a linker moiety as mentioned above in a way which does not abrogate either of the individual binding interactions (ie, to either of the two proteins). The resultant CID can be readily evaluated to confirm retention of suitable binding behavior.

To illustrate this approach we have designed a CID based on an HIV protease inhibitor and a camptothecin analog to bind to the HIV protease and translocate it into the nucleus. The incorrect compartmentalization of the protease resulting from the translocation is aimed at effectively inactivating the HIV protease. Such CIDs may bind to the protease active site and inhibit its enzymatic activity as do other HIV protease inhibitor molecules. But whether they do so or not, these CIDs are designed to abrogate protease activity by translocation to an incorrect cellular compartment.

In our illustrative approach, S-10-hydroxycamptothecin (3) and other analogs of camptothecin are obtained by known procedures. See e.g. Kingsbury et al, 1991, J. Med. Chem., 34(1), 98-107. See also, Luzzio et al, Eur. Pat. Appl. EP 540099. Hydroxycamptothecin may be linked to an HIV protease inhibitor (2)(see Lam et al and WO 94/08977, both supra, to form the CID (1) 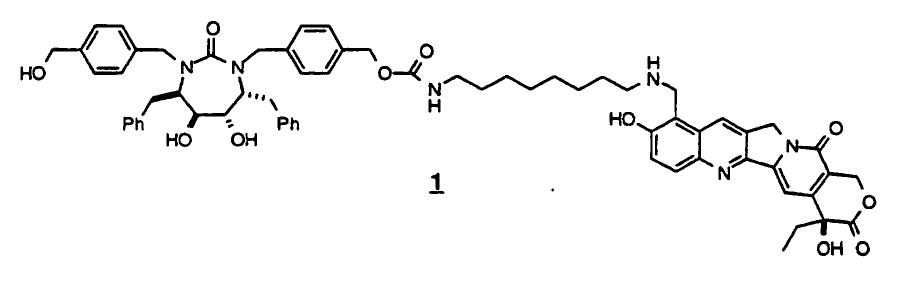 as depicted schematically below (see Kingsbury et al):

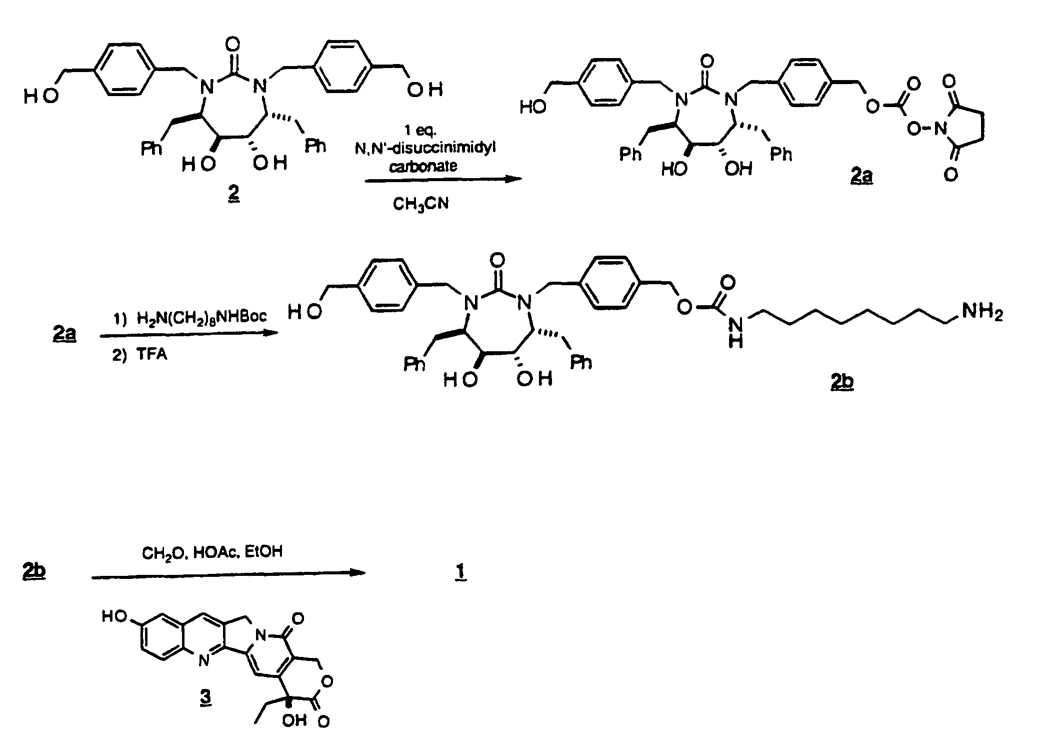

### Example 2

The approach of Example 1 is extendable to other cytoplasmic targets, e.g. zap70, to illustrate the targeting of a signal transduction mediator. While some groups are actively searching for compounds which bind to and specifically inhibit zap70, a CID of this invention which contains a zap70 binding molecule (even one which alone does not inhibit zap70 interactions or biological activity) linked to a nuclear targeting moiety such as a camptothecin moiety or the like or a protesome targeting moiety (see below) would translocate the target to an incorrect compartment, i.e. a cellular location, inconsistent with its normal biological functioning, or to the proteosome where it can be removed from the system by degradation.

### Example 3

An additional illustrative example involves the targeting of a cellular protein or component of virus such as HIV to proteosomal degradation pathways using CIDs. These CIDs have as one component, molecules known (or selected) to bind to viral proteins such as AZT and as a second component a molecule that binds to proteosome components such as the LMP7, LMP2 (Martinez and Monoco, Nature 353:664, 1991) or other components responsible at least in part for proteosome function and substrate specificity (Gaszynska,M. et al Nature 365,264,1993; A. Skiyama et al FEBS-Lett.343: 85-88,1994; and Shimbara et. al. J Biochem 115:257,1994). As in the other embodiments of this invention the binding compounds may be selected from previously known compounds which are known or thought to possess the desired binding properties, or may be selected using conventional or other binding assays from collections of compounds screened against the protein of interest (expressed for instance using conventional methods and materials). These CIDs are designed to induce the physical proximity of the targeted viral or cellular proteins to the proteosome, thereby resulting in the rapid destruction of the cellular or viral protein.

Thus, molecules that bind to the proteosome may be identified by screening of collections of compounds or by a variety of methods described above. Compounds may also be so selected from combinatorial libraries or from the store of previously known compounds which are capable of binding to essential HIV proteins, including AZT and analogs thereof and any of the numerous reported molecules that bind to the HIV protease. A compound which binds to a proteosome component is then covalently linked to one of the compounds capable of binding to the targeted HIV component.

The efficiency of the CIDs in inducing dimerization may be tested using the cell based assay described above. This method allows for comparative evaluation of modifications in CID design, including modifications to binding molecules, linker moiety and specific linkages. Following confirmation of desired activity in the induction of dimerization and activation of the zeta chain chimera, the CIDs may be tested for the intended biological activity, e.g. the ability to rid cultured cells of the target protein, using assays such as western blotting and other established methods or bioassays.

### Example 4

A further illustrative modification involves selecting a compound capable of binding to a class of proteins called E3 enzymes (see Ciechanover, 1994, Cell 79:13-21 and references cited therein). These cause proteins to which they bind to be ubiquitinated and therefore targeted for protein degradation. An example of a protein that acts by this principle is the E6 protein of the papilloma virus. It binds to an E3 protein called E6AP (E6-associated protein). E6 also binds to p53, p53 being brought in the close proximity of E6AP and E3 ligase causes it to be ubiquitinated and therefore degraded.

CIDs of this aspect of the invention are designed to contain a moiety selected for its ability to bind to an E3 enzyme covalently linked to a moiety selected to bind to a cellular, viral or other protein to be removed (e.g. HIV protease, zap 70, etc.). Binding of the CID to the targeted protein is intended to result in ubiquitination and therefore degradation of the targeted protein by the cell.

### Example 5: In vitro competitive binding assay for binding compounds for use in preparing CIDs

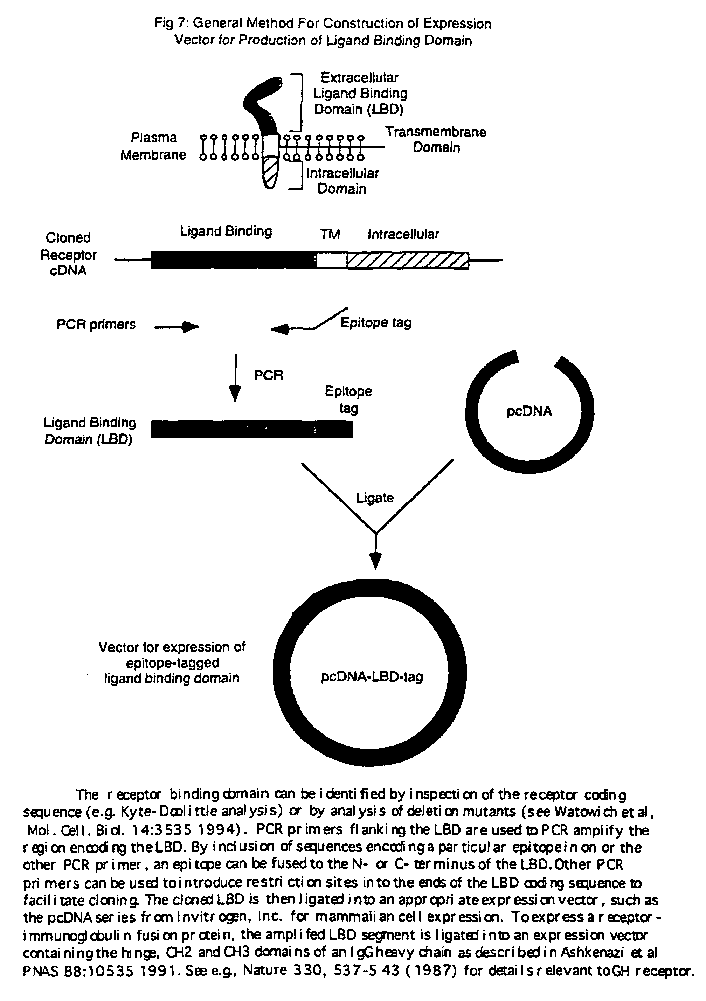

The extracellular ligand binding domain may be expressed and purified using the cloned receptor cDNA. Identification of the receptor extracellular domain can be done by performing a Kyte-Doolittle analysis on the coding sequence. In the case of cytokine and growth factor receptors, the extracellular domain is N-terminal of the transmembrane-spanning (TM) domain. The TM domain marks the end of the ligand binding domain and in the Kyte-Doolittle profile is demarked by a high hydrophobicity index over a span of between 20-30 amino acids. For an example of the Kyte-Doolittle analysis of the EPO-receptor see US Patent No. 5,278,065. See also US Patent No. 5,292,654 (mutant EPO-R). To produce the ligand binding domain of a receptor, the cDNA encoding the extracellular domain is cloned into an appropriate expression vector such as pET11a (Invitrogen) for E. coli, pVL1393 (Invitrogen) for insect cells, or pcDNA (Invitrogen) for mammalian cells. A stop codon is introduced at/before the first amino acid of the TM domain. When this so-called soluble receptor is expressed in yeast, insect cells or mammalian cells, the protein is secreted into the cell culture medium (see Kikuchi et al J. Immunol. Methods 167:289 1994). Alternatively, when the ligand binding domain is expressed in E. coli, the soluble receptor collects in the periplasmic space (see Cunningham et al Science 254: 821 1991). To facilitate purification and binding assays the extracellular domain may be expressed fused to an epitope tag such as the epitope for the anti-myc antibody 9E10 or the "Flag" epitope (IBI) (see Kolodziej and Young, Methods Enzymol 194: 508 (1991)). Alternatively, the ligand binding domain may be expressed fused to the heavy chain of an immunoglobulin as described in (Ashkenazi et al PNAS 88:10535 1991). The ligand binding domain can be expressed in E. coli, yeast, insect cells, mammalian cells or produced using an in vitro transcription/ translation system (Promega). Expression in mammalian cells can be accomplished using transient expression or by stable selection of clones using a selectable drug such as G418. For details of expression systems see Goeddel (ed.) Methods Enzymol vol 185 1990). See also, Fig. 7:

Purification of the expressed protein can be accomplished by standard chromatographic methods, by ligand affinity chromatography or by means of the fusion partner such as an antibody epitope or immunoglobulin heavy chain.

To assay for compounds that block ligand-receptor interactions, the purified ligand binding domain is first immobilized in a microtiter dish and mixed with a test compound and radiolabeled-ligand. Typically the ligand is iodinated such as in Pennica et al Biochemistry 31:1134 1992. After a suitable incubation time, the wells are washed with buffer and the bound ligand is determined by scintillation or gamma counting. Compounds that interfere with binding of the ligand are detected by a reduction in radioactivity bound to the plate. The ligand binding domain can be engineered to facilitate several aspects of the assay. For example, if the receptor ligand binding domain is expressed as a fusion protein to an immunoglobulin heavy chain, the protein can be bound to the microtiter plate via an antibody to the heavy chain constant region. Alternatively, the assay can be done in solution then the bound and unbound ligand separated by immunoprecipitation using protein-A sepharose or Pansorbin (Calbiochem) see Pennica et al Biochemistry 31:1134 1992. In addition, amino acid substitutions can be introduced into the ligand to prevent dimerization of the receptor (see Fuh et al Science 256:1677 1992). This will make it easier to detect organic small molecules that interfere with ligand binding.

Other binding assay configurations may be advantageous. For example one may attach the ligand to a plate and then incubate the test compound in solution with the soluble receptor. After a suitable time, the wells are washed and the amount bound receptor is detected. Detection can be afforded by direct radiolabeling of the receptor or via some tag on the receptor. For example, ELISA through an epitope tag, ELISA via a non-interfering epitope of the receptor or via biotin that was used to label the receptor. An alternative assay utilizes the BIAcore where the ligand is immobilized on the flow cell ("chip") and binding of ligand in the absence or presence of test compound is measured (see Corcoran et al Eur. J. Biochem. 223:831 1994).

### Example 6: Identification of receptor binding molecules from synthetic molecular diversity libraries

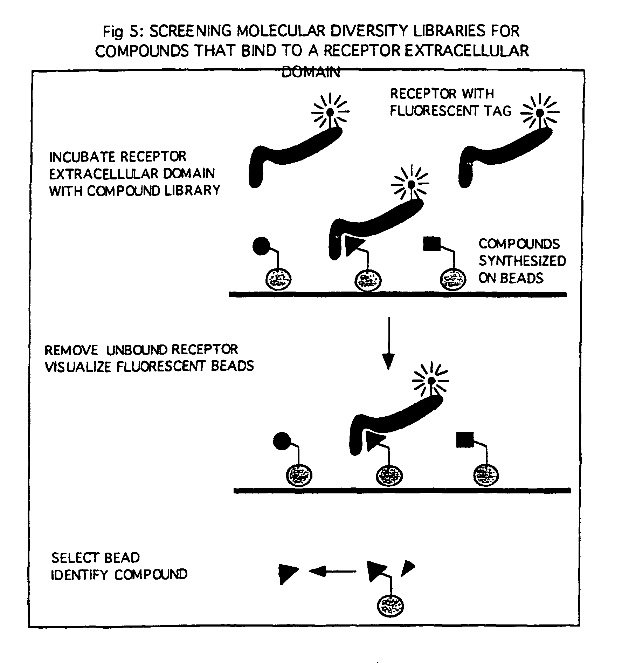

Novel ligands may also be identified using synthetic combinatorial libraries immobilized on beads, (Gordon, E.M., Barrett, R.W., Dower, W.J., Fodor, S.P. and Gallop, M.A., "Applications of combinatorial technologies to drug discovery. 2. Combinatorial organic synthesis, library screening strategies, and future directions", J. Med. Chem., 37:1385-1401 (1994)) each of which contains a unique compound. Using known methods and materials, one can synthesize libraries of millions of peptide and non-peptide ligands. To screen the library, the purified receptor extracellular domain is labeled then incubated with the beads in an appropriate buffer. After washing the mixture, beads that have bound the receptor are identified. The selected beads are isolated, and the structure of the compound on the bead is determined (See Figure 5). Various materials and methods are known in the art which are suitable for labeling the receptor so that the bound bead can be detected. These include labeling the receptor using a fluorescent molecule, biotin or an epitope tag fused to the domain. Visualization can be accomplished by fluorescence microscopy or an enzyme-linked assay using a substrate that makes the bead with bound receptor observable. Immobilized combinatorial libraries have been used, for instance, to identify ligands that bind to the Src SH3 domain (Yu, H, Chen, J.K., Feng, S, Dalgarno, D.C., Brauer, A.W., and Schreiber, S.L., "Structural basis for the binding of proline-rich peptides to SH3 domains", Cell, 76:933-945 (1994)).

### Example 7: Antagonism of ligand-mediated cellular activation

Aggregation of the intracellular domain of T cell zeta chain activates IL-2 production in T cells (Irving, B.A., and Weiss, A., "The cytoplasmic domain of the T cell receptor zeta chain is sufficient to couple to receptor-associated signal transduction pathways", Cell, 64:891-901 (1991)). Cell lines may be established in which T cell signal transduction and IL-2 production (or the production of a product encoded by a reporter gene under NFAT transcriptional control) is stimulated by addition to the medium in which the cells are being maintained of a growth factor such as EPO. In such an engineered cell line, the ligand dimerizes its receptor, thereby aggregating the zeta subunit intracellular domain and activating NFAT-controlled transcription.

A receptor chimera is constructed by PCR or by site-specific deletion mutagenesis to encode the receptor ligand binding (extracellular domain) fused to the transmembrane and intracellular domain of the T cell receptor zeta chain. Methods for creating such a chimeric with the zeta chain are described in Irving and Weiss, Cell 64: 891 (1991) and Spencer et al. Science 262, 1019 (1993). Additional methods on creating cytokine receptor chimeras can be found in Fuh et al Science 256:1677 (1992). A cDNA encoding the receptor-zeta chain chimera is inserted into a mammalian expression vector, such as described by Spencer et al. The receptor expression vector is introduced into Jurkat cells, a T cell line, along with an IL-2 reporter gene under the control of the NFAT. Cells stably expressing both the receptor chimera and reporter gene are selected by G418 selection and detection of receptor on the cell surface by FACS.

Test compounds are incubated with the cells in the presence of the ligand. Compounds that bind to the receptor and interfere with ligand binding (and receptor activation) will block IL-2 production. Alternative reporters for NFAT-dependent gene expression can be used, such as beta-galactosidase, alkaline phosphatase or luciferase, in place of IL-2. Appropriate controls may be performed to eliminate molecules that act non-specifically. Coupling a receptor to the zeta chain in a stable cell line provides a much more sensitive functional assay than using primary cell bioassays. This also allows one to select a more robust cell type suitable for screening natural product and chemical libraries.

Alternative cellular systems can be used such as the FDC-P1 cell line, dependent on G-CSF for growth (Fuh et al Science 256:1677 1992). Expression in FDC-P1 cells of a chimeric receptor containing a ligand-binding domain and the transmembrane and intracellular domain of the G-CSF receptor results in cells that are dependent on the ligand of interest for proliferation. Test compounds that interfere with the binding of ligand to receptor domain will block ligand-mediated cellular proliferation.

## Claims

1. A method for producing a non-peptidic agent capable of activating a cellular signal transduction pathway mediated by a polypeptide selected from a growth factor, cytokine and hormone, the method comprising:
(a) identifying a first non-peptide compound capable of selectively binding to an endogenous receptor for said polypeptide;
(b) identifying a second non-peptide compound capable of selectively binding to an endogenous receptor for said polypeptide; and
(c) covalently linking the first and second non-peptide compounds to each other to form a non-peptidic agent which is capable of selectively binding to more than one of the receptor molecules.

2. A method of claim 1 wherein the first and second compounds are the same.

3. A method of claim 1 wherein the first and second compounds are different.

4. A method of any of claims 1-3, wherein the receptor is a cell surface receptor for a cytokine, growth factor or hormone.

5. A method of claims 1-4 wherein the receptor is a receptor for EPO, G-CSF, TPO, GH, IL-2, interferon alpha, interferon beta, insulin or Trk.

6. A method of any of claims 1-5, wherein the agent binds to a cytoplasmic portion of the receptor.

7. The method of any of claims 1-5, wherein the agent binds to an extracellular portion of the receptor.

## Patentansprüche

1. Verfahren zum Erzeugen eines Nicht-Peptidwirkstoffs, der in der Lage ist, einen zellulären Signaltransductionsweg zu aktivieren, der vermittelt wird durch ein Polypeptid, ausgewählt aus einem Wachstumsfaktor, Cytokin und Hormon, wobei das Verfahren umfasst:
(a) Identifizieren einer ersten Nicht-Peptidverbindung, die in der Lage ist, selektiv an einen endogenen Rezeptor des Polypeptids zu binden;
(b) Identifizieren einer zweiten Nicht-Peptidverbindung, die in der Lage ist, selektiv an einen endogenen Rezeptor für das Polypeptid zu binden; und
(c) Kovalente Verknüpfung der ersten und zweiten Nicht-Peptidverbindungen aneinander, um ein Nicht-Peptidmittel zu bilden, welches in der Lage ist, selektiv an mehr als eines der Rezeptormoleküle zu binden.

2. Verfahren nach Anspruch 1, worin die ersten und zweiten Verbindungen die gleichen sind.

3. Verfahren nach Anspruch 1, worin die ersten und zweiten Verbindungen unterschiedlich sind.

4. Verfahren nach einem der Ansprüche 1-3, worin der Rezeptor ein Zelloberflächenrezeptor für ein Cytokin, Wachstumsfaktor oder Hormon ist.

5. Verfahren nach einem der Ansprüche 1-4, worin der Rezeptor ein Rezeptor für EPO, G-CSF, TPO, GH, IL-2, alpha-Interferon, beta-Interferon, Insulin oder Trk ist.

6. Verfahren nach einem der Ansprüche 1-5, worin der Wirkstoff an einen cytoplasmatischen Teil des Rezeptors bindet.

7. Verfahren nach einem der Ansprüche 1-5, worin der Wirkstoff an einen extrazellulären Teil des Rezeptors bindet.

## Revendications

1. Procédé d'obtention d'un agent non peptidique capable d'activer un trajet de transduction de signal cellulaire à médiation par un polypeptide choisi parmi un facteur de croissance, une cytokine et une hormone, le procédé comprenant :
(a) l'identification d'un premier composé non peptide capable d'être lié sélectivement à un récepteur endogène pour ledit polypeptide ;
(b) l'identification d'un second composé non peptide capable d'être lié sélectivement à un récepteur endogène pour ledit polypeptide ; et
(c) la liaison par covalence des premier et second composés non peptides l'un à l'autre pour former un agent non peptidique qui est capable d'être lié sélectivement à plus d'une des molécules de récepteur.

2. Procédé selon la revendication 1, dans lequel les premier et deuxième composés sont identiques.

3. Procédé selon la revendication 1, dans lequel les premier et deuxième composés sont différents.

4. Procédé selon l'une quelconque des revendications 1 à 3, dans lequel le récepteur est un récepteur de surface de cellule pour une cytokine, un facteur de croissance ou une hormone.

5. Procédé selon l'une quelconque des revendications 1 à 4, dans lequel le récepteur est un récepteur pour EPO, G-CSF, TPPO, GH, IL-2, l'interféron alpha, l'interféron bêta, l'insuline ou Trk.

6. Procédé selon l'une quelconque des revendications 1 à 5, dans lequel l'agent se lie à une partie cytoplasmique du récepteur.

7. Procédé selon l'une quelconque des revendications 1 à 5, dans lequel l'agent se fixe à une partie extracellulaire du récepteur.
